# EUROPÄISCHE PATENTSCHRIFT

(11) **EP 1 799 283 B1**
(45) Veröffentlichungstag und Bekanntmachung des Hinweises auf die Patenterteilung: **27.03.2013**
(21) Anmeldenummer: 05787484.4
(22) Anmeldetag: 10.09.2005
(51) Int. Cl.: A61M 1/16

(54) **BLUTBEHANDLUNGSGERÄT MIT STANGENARTIGEM MITTEL ZUR HALTERUNG VON GEGENSTÄNDEN**
BLOOD TREATMENT DEVICE COMPRISING ROD-SHAPED MEANS FOR HOLDING ARTICLES
APPAREIL DE TRAITEMENT SANGUIN POURVU D'UN MOYEN, EN FORME DE TIGE, SERVANT A MAINTENIR DES OBJETS

(30) Priorität: 06.10.2004 DE 102004048911
(43) Veröffentlichungstag der Anmeldung: 27.06.2007
(60) Teilanmeldung: 12002406.2
(73) Patentinhaber: Fresenius Medical Care Deutschland GmbH, 61352 Bad Homburg (DE)
(72) Erfinder: BREHM, Winfried, 97461 Hofheim (DE)
(74) Vertreter: Ziermann, Oliver
(86) Internationale Anmeldenummer: PCT/EP2005/009766
(87) Internationale Veröffentlichungsnummer: WO 2006/037429

(56) Entgegenhaltungen:
- US-A- 4 585 436
- US-A- 5 653 681
- US-A- 5 895 571
- US-A1- 2004 154 966

## Beschreibung

Die Erfindung betrifft das Gebiet von Blutbehandlungsgeräten nach dem Oberbegriff von Anspruch 1.

Zur extrakorporalen Blutbehandlung werden unterschiedlichste Geräte eingesetzt. Ein derartiges Blutbehandlungsgerät weist ein Blutbehandiungsmodul und in vielen Fällen noch weitere Module auf. Das Blutbehandlungsmodul ist dabei geeignet, Komponenten eines extrakorporalen Blutkreislaufes aufzunehmen. Mit Hilfe eines solchen extrakorporalen Blutkreislaufes wird meist in Form von für den Einmalgebrauch ausgebildeten Artikeln Blut von einem Patienten über eine Blutzuführleitung zu einem Blutbehandlungselement und von dort über eine Blutrückführleitung zurück zum Patienten zirkuliert. Bei dem Blutbehandlungselement kann es sich zum Beispiel um einen durch eine semipermeable Membran in zwei Kammern geteilten Hämofilter oder Hämodialysator zur Nierenersatzbehandlung handeln, dessen eine Kammer von dem extrakorporalen Blut durchflossen wird. Die andere Kammer dient in diesem Fall zur Abführung von zu entfernenden Stoffen und/oder Flüssigkeit und kann im Falle der Hämodialyse von einer Reinigungsflüssigkeit durchflossen werden.

Es sind aber auch andere Blutbehandlungselemente denkbar wie z.B. ein Oxygenator zur künstlichen Beatmung oder ein Blutadsorptionselement zur Entfernung bestimmter Stoffe aus dem extrakorporalen Blut durch Adsorption an entsprechend präparierten Obefflächenelementen.

Das Blutbehandlungsmodul des Blutbehandlungsgerätes weist zu diesem Zweck Sensoren und/oder Aktoren wie Pumpen und/oder Ventile zur Aufnahme der Komponenten des extrakorporalen Blutkreislaufs auf, um die Blutbehandlung entsprechend steuern und überwachen zu können.

Bei derartigen Blütbehandlungen kann es erforderlich sein, in Behältern, insbesondere Beuteln, befindliche Lösungen zur Verwendung während der Blutbehandlung bereit zu halten. Bei derartigen Lösungen kann es sich um Lösungen handeln, die für die Blutbehandlung selbst unmittelbar benötigt werden, wie zum Beispiel Dialyselösung im Fall der Hämodialysebehandlung oder Substituatlösung im Fall der Hämofiltrationsbehandlung. Es kann sich aber auch um Behälter beziehungsweise Lösungen handeln, die nur in bestimmten Situationen wie zum Beispiel beim Auftreten von Komplikationen benötigt werden. So kann es während einer Hämodialysebehandlung zu einem plötzlichen Blutdruckabfall kommen, dem mit der Infusion von physiologischer Kochsalzlösung begegnet werden kann.

Aus diesem Grund sind Blutbehandlungsgeräte verbreitet, an die eine herkömmliche Infusionsstange mit einem als sogenanntes Flaschenkreuz ausgebildeten Halterungsmittel montiert ist. Eine solche Anordnung hat den Vorteil, dass neben dem Blutbehandlungsgerät nicht noch ein getrennter Infusionsständer bereitgestellt werden muss. Die am Blutbehandlungsgerät befestigte Infusionsstange kann auf diese Weise gleichzeitig mit dem Blutbehandlungsgerät, das meist auf als Rollen ausgebildeten Standelementen gelagert ist, zur Anpassung an eine veränderliche Lage des Patienten verschoben werden.

Da die Blutbehandlungsgeräte eine beachtliche Ausdehnung aufweisen können, hat sich diese herkömmliche Anordnung dahingehend als nachteilig erwiesen, als dass sich in diesem Fall das Flaschenkreuz unmittelbar über dem Blutbehandlungsgerät befindet. Hierbei kann sich der Zugang zum Flaschenkreuz beim Anbringen und Abnehmen von Gegenständen als schwierig erweisen, da der kürzeste Zugang von unten nicht unmittelbar zugänglich ist.

US 4,585,436 offenbart eine Vorrichtung für die Peritonealdialyse mit einem Trägergestell für mehrere Dialysebeutel. Das Tägergestell ist auf der Oberseite der Vorrichtung für die Peritonealdialyse angebracht und besteht aus einem zwei vertikalen Trägerteilen, die mit einer Strebe verbunden sind und einer horizonal angeordneten Tragestange ("rack"), die jeweils mit den beiden vertikalen Trägerteilen verbunden ist. Auf beiden Seiten der Trägerstange können Dialysebeutel mit Halterungen aufgeschoben werden.

Die Erfindung hat sich daher die Aufgabe gestellt, ein Blutbehandlungsgerät bereit zu stellen, das einen vereinfachten Zugang zu dem zum Beispiel als Flaschenkreuz ausgebildeten Halterungsmittel ermöglicht.

Die Lösung dieser Aufgabe gelingt mit einem Blutbehandlungsgerät mit den Merkmalen des Anspruch 1. Vorteilhafte Ausgestaltungen sind Gegenstand der Unteransprüche.

Das erfindungsgemäße Blutbehandlungsgerät weist ein Gehäuse und ein Blutbehandlungsmodul auf, wobei durch die Orientierung des Gehäuse während der Benutzung des Blutbehandlungsgerätes vertikale und horizontale Richtungen definiert werden. Dabei ist ferner ein stangenartiges Mittel an dem Blutbehandlungsgerät befestigt, dessen axiale Ausdehnung zumindest eine zum Gehäuse vertikal verlaufende Komponente aufweist. Mit dem stangenartigen Mittel ist ein Halterungsmittel zur Halterung von Gegenständen verbunden. Erfindungsgemäß weist das stangenartige Mittel zumindest einen ersten Abschnitt und einen zweiten Abschnitt auf, wobei die axiale Ausdehnung im zweiten Abschnitt in eine andere Richtung als im ersten Abschnitt verläuft. Diese Anordnung ermöglicht es, das Halterungsmittel in einem Bereich des stangenartigen Mittels anzuordnen, der einen leichteren Zugang von unten gestattet Besonders vorteilhaft ist dabei eine Ausführungsform, bei der die vertikale Projektion des Schwerpunkts des Halterungsmittels neben der vertikalen Projektion des Gehäuses verläuft.

Nähere Einzelheiten der Erfindung werden anhand eines in den Zeichnungen gezeigten Ausführungsbeispieles näher erläutert. Es zeigen
Fig. 1 eine schematische Darstellung einer Ausführungsform eines erfindungsgemäßen Blutbehandlungsgerätes und
Fig. 2 die einzelnen Abschnitte des stangenartigen Mittels der in Fig. 1 gezeigten Ausführungsform.

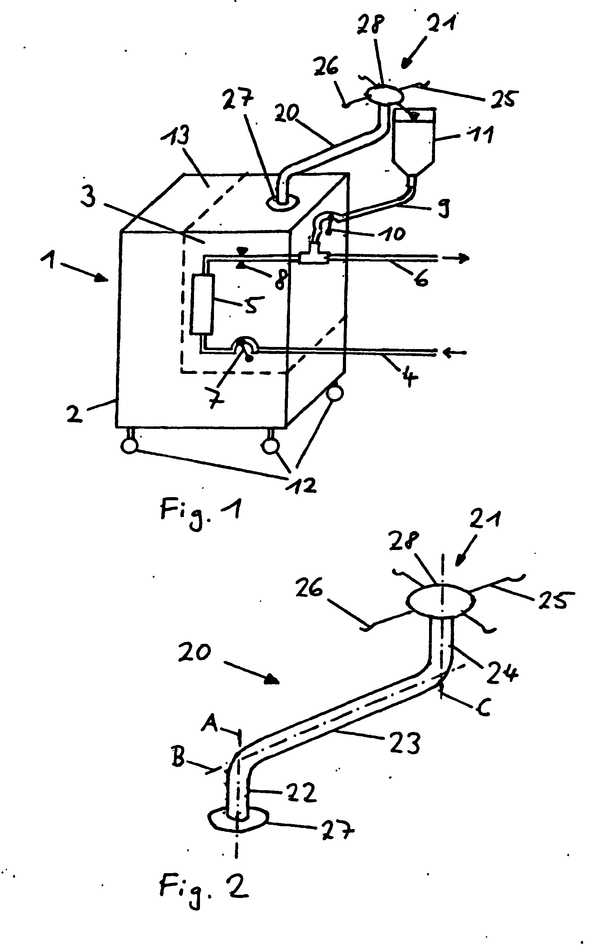

In Fig. 1 ist ein Blutbehandlungsgerät 1 mit einem Gehäuse 2 und einem Blutbehandlungsmodul 3 gezeigt. Das Blutbehandlungsmodul 3, dessen Verlauf schematisch durch die gestrichelte Linie angedeutet ist, ist geeignet, Komponenten eines extrakorporalen Kreislaufs aufzunehmen. Von diesem sind in Fig. 1 eine von einem nicht gezeigten Patienten zu einem Blutbehandlungselement 5 führende Blutzuführleitung 4 und eine von dem Blutbehandlungselement 5 zum Patienten zurückführende Blutrückführleitung 6 gezeigt. Das Blutbehandlungselement kann zum Beispiel aus einem durch eine semipermeable Membran in zwei Kammern geteilten Hämodialysator oder Hämofilter bestehen. Derartige Blutbehandlungselemente sind oft in der Form von Hohlfasermodulen ausgebildet, die Tausende von Membranfasern beinhalten, deren Inneres von dem extrakorporalen Blut durchflossen wird. Komponenten, die den Anschluss von Leitungen für weitere Flüssigkeiten wie Dialyseflüssigkeit an das Blutbehandlungselement betreffen, sowie weitere dem Fachmann geläufige Elemente eines extrakorporalen Blutkreislaufs sind der Übersichtlichkeit halber nicht in Fig. 1 gezeigt.

Die meist als Einmalartikel ausgebildeten Elemente des extrakorporalen Blutkreislaufes werden dabei mit Aktoren und Sensoren des Blutbehandlungsmoduls 3 verbunden, damit das Blutbehandlungsgerät die eigentliche Blutbehandlung steuern und überwachen kann. Von diesen Aktoren und Sensoren sind hier exemplarisch eine Blutpumpe 7 und eine venöse Klemme 8 dargestellt Mit Hilfe der Blutpumpe 7 wird das Blut im extrakorporalen Kreislauf zirkuliert. Die venöse Klemme 8 dient der Unterbrechung des Flusses im extrakorporalen Kreislauf im Falle einer Alarmsituation.

Das Gehäuse 2 weist am unteren Ende als Rollen ausgebildete Standelemente 12 auf, damit das Blutbehandlungsgerät nach Bedarf verschoben werden kann. An der oberen Begrenzungsfläche 13 des Gehäuses 2 ist ein stangenartiges Mittel 20 über eine Drehkupplung 27 befestigt. Das stangenartige Mittel 20 erstreckt sich ausgehend von einem ersten, unteren Ende axial mit einer zum Gehäuse 2 vertikal verlaufenden Komponente, wobei dass Gehäuse 2 durch seine Aufstellung für die Benutzung des Blutbehandlungsgerätes vertikale und horizontale Richtungen definiert. Am zweiten, oberen Ende des stangenartigen Mittels 20 befindet sind ein Halterungsmittel 21. Dieses besteht in der in Fig. 1 gezeigten Ausführungsform aus kreuzartig angeordneten Stangen 25, die eine zum -Gehäuse 2 horizontale Ebene definieren. An den Enden der Stangen 25 sind Haken 26 zur Halterung von Gegenständen vorgesehen. Die Stangen 25 sind dabei an einem an dem stangenartigen Mittel 20 befestigten Halterungsblock 28 befestigt.

An einem der Haken 27 ist exemplarisch ein als mit Infusionsflüssigkeit gefüllter Beutel ausgebildeter Gegenstand 11 aufgehängt gezeigt. Der Beutel 11 ist mit einer Verbindungsleitung 9 mit der Blutrückführleitung 6 über ein T-Verbindungsstück verbunden. In dieser Ausführungsform führt die Verbindungsleitung 9 ferner über eine an dem Blutbehandlungsmodul 3 vorgesehene Infusionspumpe 10, damit eine benötigte Infusion durch das Blutbehandlungsgerät 1 gesteuert werden kann.

In Fig. 2 ist das stangenartige Mittel 20 im Detail gezeigt. Es besteht aus drei Abschnitten 22, 23 und 24. Der an der Drehkupplung 27 befestigte erste Abschnitt 22 weist eine axiale Ausdehnung A auf, die vertikal zum Gehäuse verläuft. An diesen ersten Abschnitt 22 schließt sich ein zweiter Abschnitt 23 an, dessen axiale Ausdehnung B sich in andere Richtung als die des ersten Abschnittes erstreckt. In dieser Ausführungsform weist die axiale Ausdehung B sowohl eine horizontale als auch eine vertikale Komponente auf. Auf diese Weise wird erreicht, dass die senkrechte Projektion des an dem stangenartigen Mittel 20 befestigten Halterungsmittels 21 bezogen auf die Befestigung des ersten Abschnitts 22 am Gehäuse 2 horizontal verschoben ist. In der in Fig. 1 gezeigten Ausführungsform verläuft die vertikale Projektion sogar neben der vertikalen Projektion des Gehäuses 2. Das Halterungsmittel 21 ist hierdurch leichter von unten zugänglich.

In der in den Zeichnungen gezeigten Ausführungsform schließt sich nun noch ein dritter Abschnitt 24 an den zweiten Abschnitt 23 des stangenartigen Mittels 20 an, dessen axiale Ausdehnung C parallel zur axialen Ausdehnung des ersten Abschnittes erfolgt, d.h. in dieser Ausführungsform ergibt sich ein gekröpfter Verlauf des stangenartigen Mittels 20.

Die Drehkupplung 27 ermöglicht ein Schwenken des gesamten stangenartigen Mittels 20 über die obere Begrenzungsfläche 13 des Blutbehandlungsgerätes 1, sollte dies zum Beispiel beim Transport des Blutbehandlungsgerätes 1 notwendig werden oder sich im Einzelfall während einer Blutbehandlung zum Beispiel aus Platzgründen als sinnvoll erweisen.

Das stangenartige Mittel 20 beziehungsweise seine einzelnen Abschnitte können einstückig oder aus mehreren zusammengesteckten Komponenten ausgebildet sein. Dabei kann wie bei einem Baukastensystem vorgesehen sein, durch verschiedene Komponenten unterschiedliche vertikale und horizontale Verläufe des stangenartigen Mittels 20 je nach Bedarf zu realisieren.

Die Gestaltung des erfindungsgemäßen Blutbehandlungsgerätes ermöglicht damit einen vereinfachten Zugang zu dem Halterungsmittel für zum Beispiel Beutel mit medizinischen Lösungen, ohne dass auf die Vorteile eines konventionellen Gerätes verzichtet werden muss, bei dem eine vertikale Infusionsstange an dem Gehäuse des Blutbehandlungsgerätes befestigt ist

## Patentansprüche

1. Blutbehandlungsgerät (1) mit einem Gehäuse (2) und einem Blutbehandlungsmodul (3), wobei durch die Orientierung des Gehäuses während der Benutzung des Bhubehandlungsgerätes vertikale und horizontale Richtungen definiert werden, mit einem an einer oberen Begrenzungsfläche (13) des Gehäuses (2) befestigten stangenartigen Mittel (20), dessen axiale Ausdehnung zumindest eine zum Gehäuse vertikal verlaufende Komponente aufweist, und mit einem mit dem stangenartigen Mittel (20) verbundenen Halterungsmittel (21) zur Halterung von Gegenständen, wobei das stangenartige Mittel (20) zumindest einen ersten Abschnitt (22) und einen zweiten Abschnitt (23) aufweist, wobei die axiale Ausdehnung im zweiten Abschnitt (23) in eine andere Richtung als im ersten Abschnitt (22) verläuft **dadurch gekennzeichnet, dass** das staugenartige Mittel (20) um die Achse des ersten Abschnittes (22) drehbar an dem Blutbehandlungsgerät (1) befestigt ist.

2. Blutbehandlungsgerät nach einem der vorhergehenden Ansprüche, **dadurch gekennzeichnet, dass** der zweite Abschnitt (23) zwischen dem ersten (22) und einem dritten Abschnitt (24) verläuft, wobei auch die axiale Ausdehnung des dritten Abschnitts (24) in eine andere Richtung als die des zweiten Abschnitts (23) verläuft.

3. Blutbehandlungsgerät nach Anspruch 2, **dadurch gekennzeichnet, dass** die axiale Ausdehnung des ersten (22) und dritten Bereiches (24) in parallele Richtungen verlaufen.

4. Blutbehandlungsgerät nach Anspruch 3, **dadurch gekennzeichnet, dass** die axiale Ausdehnung des ersten (22) und dritten Bereiches (24) in zum Gehäuse (2) vertikaler Richtung verlaufen.

5. Blutbehandlungsgerät nach einem der vorhergehenden Ansprüche, **dadurch gekennzeichnet, dass** die vertikale Projektion des Schwerpunkts des Halterungsmittels (21) neben der vertikalen Projektion des Gehäuses (2) verläuft.

6. Blutbehandlungsgerät nach einem der vorhergehenden Ansprüche, **dadurch gekennzeichnet, dass** das Haltenmgsmittel (21) haken- und/oder stangenartige Befestigungsmittel (25, 26) umfasst.

7. Blutbehandlungsgerät nach Anspruch 6, **dadurch gekennzeichnet, dass** das Befestigungsmittel aus kreuzartig angeordneten Stangen (25) besteht, die eine zum Gehäuse (2) horizontale Ebene definieren.

8. Blutbehandlungsgerät nach Anspruch 7, **dadurch gekennzeichnet, dass** an den Enden der Stangen (25) Haken (26) zur Halterung der Behälter mit medizinischen Lösungen, insbesondere Beutel (11) mit Infusions- und/oder Dialyselösung, vorgesehen sind.

9. Blutbehandhungsgerät nach einem der vorhergehenden Ansprüche, **dadurch gekennzeichnet, dass** das Blutbehandlungsmodul (3) geeignet ist, Komponenten eines extrakorporalen Blutkreislaufes, der eine von einem Patienten zu einem Blutbehandlungselement (5) führende Blutzuführleitung (4), ein Blutbehandlungselement (5) sowie eine von dem Blutbehandlungselement (5) zum Patienten zurückführende Blutrückführleitung (6) umfasst, aufzunehmen.

10. Blutbehandlungsgerät nach Anspruch 9, **dadurch gekennzeichnet, dass** es sich bei dem Blutbehandhungselement um einen durch eine semipermeable Membran in zwei Kammern geteilten Hämofilter oder Hämodialysator handelt, deren eine Kammer mit der Blutzuführleitung (4) und der Blutrörkführleitung (6) verbunden ist.

11. Blutbehandlungsgerät nach Anspruch 9, **dadurch gekennzeichnet, dass** das Blutbehandlungsmodul (3) Aktoren und/oder Sensoren (7, 8) zur Aufnahme der Komponenten des extrakorporalen Blutkreislaufs aufweist.

12. Blutbehandlungsgerät nach Anspruch 9, 10 oder 11, **dadurch gekennzeichnet, dass** das Blutbehandlungsmodul geeignet ist, eine den gehalterten Behälter mit dem extrakorporalen Kreislauf verbindende flüssigkeitsführende Leitung (9) aufzunehmen.

13. Blutbehandlungsgerät nach Anspruch 12, **dadurch gekennzeichnet, dass** das Blutbehandlungsmodul (3) zur Aufnahme der verbindenden flüssigkeitsführenden Leitung (9) Aktoren und/oder Sensoren (10) aufweist.

## Claims

1. A blood treatment device (1) having a housing (2) and a blood treatment module (3), wherein vertical and horizontal directions are defined by the orientation of the housing during the use of the blood treatment device, having a rod-shaped means (20) attached to an upper bordering surface (13) of the housing (2), its axial extent having at least one component running vertically to the housing, and having a holding device (21) connected to the rod-shaped means (20) for holding objects, the rod-shaped means (20) having at least one first section (22) and one second section (23), wherein the axial extent in the second section (23) runs in a different direction than in the first section (22),
**characterized in that**
the rod-shaped means (20) are attached to the blood treatment device (1) in such a way as to permit rotation about the axis of the first section (22).

2. The blood treatment device according to any one of the preceding claims,
**characterized in that**
the second section (23) runs between the first section (22) and a third section (24), wherein the axial extent of the third section (24) also runs in a different direction than the second section (23).

3. The blood treatment device according to Claim 2,
**characterized in that**
the axial extent of the first area (22) and that of the third area (24) run in parallel directions.

4. The blood treatment device according to Claim 3,
**characterized in that**
the axial extent of the first area (22) and that of the third area (24) run in a direction vertical to the housing (2).

5. The blood treatment device according to any one of the preceding claims,
**characterized in that**
the vertical projection of the center point of the holding means (21) runs next to the vertical projection of the housing (2).

6. The blood treatment device according to any one of the preceding claims,
**characterized in that**
the holding means (21) include hook-shaped and/or rod-shaped fastening means (25, 26).

7. The blood treatment device according to Claim 6,
**characterized in that**
the fastening means consist of rods (25) arranged in a cross, defining a horizontal plane in relation to the housing (2).

8. The blood treatment device according to Claim 7,
**characterized in that**
hooks (26) for holding the containers of medical solutions, in particular bags (11) of infusion solution and/or dialysis solution, are provided at the ends of the rods (25).

9. The blood treatment device according to any one of the preceding claims,
**characterized in that**
the blood treatment module (3) is suitable for accommodating components of an extracorporeal blood circulation, comprising a blood supply line (4) leading from a patient to a blood treatment element (5), a blood treatment element (5) and a blood return line (6) leading from the blood treatment element (5) back to the patient.

10. The blood treatment device according to Claim 9,
**characterized in that**
the blood treatment element is a hemofilter or hemodialyzer that is divided by a semipermeable membrane into two chambers, one chamber of which is connected to the blood supply line (4) and the other of which is connected to the blood return line (6).

11. The blood treatment device according to Claim 9,
**characterized in that**
the blood treatment module (3) has actuators and/or sensors (7, 8) for receiving the components of the extracorporeal blood circulation.

12. The blood treatment device according to Claims 9, 10 or 11,
**characterized in that**
the blood treatment module is suitable for accommodating a liquid-carrying line (9) connecting the container being held to the extracorporeal circulation.

13. The blood treatment device according to Claim 12,
**characterized in that**
the blood treatment module (3) has actuators and/or sensors (10) to accommodate the liquid-carrying line (9) that is to be connected.

## Revendications

1. Dispositif de traitement du sang (1), comprenant un boitier (2) et un module de traitement du sang (3), dans lequel, par l'orientation du boitier pendant l'utilisation du dispositif de traitement du sang, des directions verticales et horizontales sont définies, comprenant un moyen (20) en forme de tige fixé sur une surface de délimitation supérieure (13) du boitier (2), dont la dilatation axiale présente au moins une composante au parcours vertical par rapport au boitier, et un moyen de fixation (21) relié au moyen (20) en forme de tige pour fixer des objets, sachant que le moyen (20) en forme de tige présente au moins un premier tronçon (22) et un deuxième tronçon (23), sachant que la dilatation axiale dans le deuxième tronçon (23) part dans une autre direction que dans le premier tronçon (22), **caractérisé en ce que** le moyen (20) en forme de tige est fixé sur le dispositif de traitement du sang (1) en tournant sur l'axe du premier tronçon (22).

2. Dispositif de traitement du sang selon la revendication 1, **caractérisé en ce que** le deuxième tronçon (23) passe entre le premier (22) et un troisième tronçon (24), sachant que la dilatation axiale du troisième tronçon (24) part également dans une autre direction que celle du deuxième tronçon (23).

3. Dispositif de traitement du sang selon la revendication 2, **caractérisé en ce que** les dilatations axiales du premier (22) et du troisième tronçon (24) partent dans des directions parallèles.

4. Dispositif de traitement du sang selon la revendication 3, **caractérisé en ce que** les dilatations axiales du premier (22) et du troisième tronçon (24) partent dans une direction verticale par rapport au boitier (2).

5. Dispositif de traitement du sang selon l'une des revendications précédentes, **caractérisé en ce que** la projection verticale du centre de gravité du moyen de fixation (21) passe à côté de la projection verticale du boitier (2).

6. Dispositif de traitement du sang selon l'une des revendications précédentes, **caractérisé en ce que** le moyen de fixation (21) comprend des moyens de fixation (25, 26) en forme de crochet et/ou de tige.

7. Dispositif de traitement du sang selon la revendication 6, **caractérisé en ce que** le moyen de fixation est composé de tiges (25) agencées en forme de croix qui définissent un plan horizontal par rapport au boitier (2).

8. Dispositif de traitement du sang selon la revendication 7, **caractérisé en ce que** des crochets (26) sont prévus sur les extrémités des tiges (25) pour fixer les contenants avec des solutions médicales, en particulier des poches (11) comprenant une solution de perfusion et/ou de dialyse.

9. Dispositif de traitement du sang selon l'une des revendications précédentes, **caractérisé en ce que** le module de traitement du sang (3) est approprié pour recevoir des composantes d'une circulation sanguine extracorporelle qui comprend une ligne d'admission de sang (4) allant d'un patient vers un élément de traitement du sang (5), un élément de traitement du sang (5) ainsi qu'une ligne de retour du sang (6) partant de l'élément de traitement du sang (5) vers le patient.

10. Dispositif de traitement du sang selon la revendication 9, **caractérisé en ce qu'**il s'agit pour l'élément de traitement du sang d'un hémofiltre ou d'un hémodialyseur divisé en deux chambres par une membrane semi-perméable, dont une chambre est reliée à la ligne d'admission de sang (4) et la ligne de retour du sang (6).

11. Dispositif de traitement du sang selon la revendication 9, **caractérisé en ce que** le module de traitement du sang (3) comprend des actionneurs et/ou des capteurs (7, 8) pour recevoir les composantes de la circulation sanguine extracorporelle.

12. Dispositif de traitement du sang selon la revendication 9, 10 ou 11, **caractérisé en ce que** le module de traitement du sang est approprié pour recevoir une ligne conduisant le liquide (9) reliant le récipient fixé à la circulation sanguine extracorporelle.

13. Dispositif de traitement du sang selon la revendication 12, **caractérisé en ce que** le module de traitement du sang (3) comprend des actionneurs et/ou des capteurs (10) pour recevoir la ligne conduisant le liquide (9) effectuant la liaison.
